# EUROPEAN PATENT SPECIFICATION

(11) **EP 2 765 093 B1**
(45) Date of publication and mention of the grant of the patent: **07.12.2016**
(21) Application number: 14167274.1
(22) Date of filing: 13.04.2009
(51) Int. Cl.: B65D 83/08, B65D 81/28, A61B 42/40

(54) **GLOVE PACKAGING HAVING ANTIMICROBIAL BARRIER**
HANDSCHUHVERPACKUNG MIT ANTIMIKROBIELLER SPERRE
EMBALLAGE POUR GANTS PRÉSENTANT UNE BARRIÈRE ANTIMICROBIENNE

(30) Priority: 18.04.2008 US 148448
(43) Date of publication of application: 13.08.2014
(62) Divisional of application: 09731868.7
(73) Proprietor: Medline Industries, Inc., Mundelein, IL 60060 (US)
(72) Inventor: Yao, Min, Shangai (CN); Amdur, Samuel, T., H., III, Libertyville, IL Illinois 60048 (US)
(74) Representative: Carter, Stephen John

(56) References cited:
- WO-A1-2008/136721
- US-A- 5 501 323
- US-A1- 2002 043 537

## Description

### FIELD OF THE INVENTION

The present invention relates generally to packaging for gloves. More particularly, the present invention relates to packaging for gloves having an antimicrobial barrier for protecting the gloves from contamination from microorganisms and other undesirable materials or contaminants and methods for making the packaging.

### BACKGROUND OF THE INVENTION

Gloves are widely used as a protective measure and have become mandatory in many industries and nearly all medical and surgical settings. In particular, disposable gloves are required as a means for protecting medical and surgical staff from coming into contact with bodily fluids during surgical procedures, medical examinations, laboratory testing and other medical procedures. Disposable gloves have traditionally been made of rubber materials such as latex, thermoplastic materials such as vinyl, and other natural and synthetic materials.

Many gloves are,provided in packaging having a cavity for holding the gloves. The packaging includes an opening for removing the gloves from the packaging. The opening is typically revealed by removing a perforated portion of the packaging to access the gloves. Once the perforated portion of the packaging is removed to reveal the opening, the gloves are exposed to the ambient environment. As the ambient environment may contain microorganisms, pathogens, small airborne particles of dust and debris and other air contaminants, the gloves contained in the packaging may be exposed to undesirable materials or contaminants that may contaminate the gloves while they are in the packaging.

Thus, there exists a need for a glove packaging that includes a barrier to protect gloves contained within the glove packaging from microorganisms, airborne particles and other materials or contaminants that may contaminate the gloves prior to removal from the packaging. Preferably, the barrier can also destroy any microorganisms, pathogens or other materials or contaminants that come in contact with the barrier to further reduce the possibility of contamination.

WO 2008/136721 describes a disposable container for tissues. US2002/043537 describes another example of a dispenser for tissues. US 5501323 describes a sealed dispenser system for rubber or latex gloves.

In one aspect the invention provides a container for disposable medical gloves as set forth in claim 1.

In anothe aspect the invention provides a method of making a container for disposable medical gloves as set forth in claim 11.

The detailed description and Figures will describe many of the embodiments and aspects of the present concepts.

### BRIEF DESCRIPTION OF THE DRAWINGS

The foregoing and other advantages of the invention will become apparent upon reading the following detailed description and upon reference to the drawings.
FIG. 1 is a perspective view of a glove packaging according to one embodiment of the present concepts.
FIG. 2 is a top view of the glove packaging illustrating an antimicrobial barrier.
FIG. 3 is a side view of a barrier including an antimicrobial material.
FIG. 4 is a side view of another embodiment of a barrier including an antimicrobial material.

While the invention is susceptible to various modifications and alternative forms, specific embodiments have been shown by way of example in the drawings and will be described in detail herein. It should be understood, however, that the invention is not intended to be limited to the particular forms disclosed. Rather, the invention is to cover all modifications, equivalents, and alternatives falling within the scope of the claims

### DESCRIPTION OF ILLUSTRATIVE EMBODIMENTS

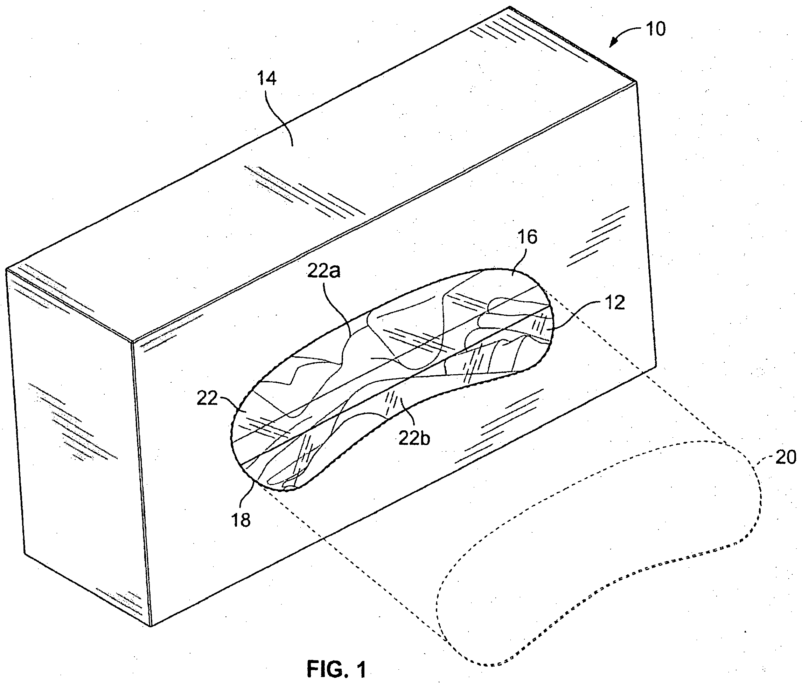

FIG. 1 illustrates a packaging 10 for gloves 12. The packaging 10 comprises a container 14 having a cavity 16 for holding the gloves 12. The container 14 includes an opening 18 for removing the gloves 12 from the container 14. The opening 18 may be in the form of different shapes, such as a circle, an oval, a square, a rectangle, or any variation of such shapes, such that a user may insert his or her hand through the opening 18 to remove one or more of the gloves 12. The opening 18 may initially be covered by a removable segment 20 that is initially formed as part of the container 14. The removable segment 20, which may be a perforated segment, is removable from the packaging 10 by a user once the packaging 10 is ready to be opened by tearing the removable segment 20 away from the packaging 10. The removable segment 20 is generally discarded after it is removed from the packaging 10. In addition to a perforated segment, the removable segment 20 may include an adhesive segment that is removable from the packaging 10.

The container 14 and removable segment 20 may be comprised of a variety of materials or combinations of materials, such as paper, plastic or fabric. The gloves 12 may include medical and/or surgical disposable gloves that arc comprised of rubber materials such as latex, thermoplastic materials such as vinyl, and other natural and synthetic materials, such as nitrile, polyvinyl chloride, polyethylene, polyisoprene, neoprene, polychloriprene, etc. The gloves 12 may include other materials, such as antimicrobial coatings and/or coatings for protecting the skin that include aloe, chamomile, vitamin(s), or combinations thereof and other suitably ingredients that may provide skin care benefits, such as moisturizing and soothing dry, irritated skin. In addition to disposable gloves, it is contemplated that other types of gloves, i.e., non-medical or non-surgical gloves, could be used with the present concepts.

The packaging 10 may also comprise a barrier 22 that covers at least a portion of the opening 18 of the container 14. The barrier 22 may be a film, a piece of paper laminated with film or any type of flexible material that is suitable for providing or acting as a barrier. The barrier 22 inhibits or prevents microorganisms, pathogens, small airborne particles of dust and debris and other air contaminants from contacting and thus contaminating the gloves 12 prior to removal from the packaging 10. Thus, the barrier 22 helps to protect the gloves 12 from being exposed to undesirable materials or contaminants while the gloves 12 arc in the packaging 10. Providing gloves 12 that are free from undesirable materials or contaminants reduces the risk that patients and healthcare workers will be exposed to such materials or contaminants and thereby reduces the opportunity to spread potentially harmful and infectious materials or contaminants. Furthermore, providing gloves 12 that are free from undesirable materials or contaminants also reduces or prevents cross-contamination that may occur between different patients.

In one embodiment, where the barrier 22 is a film, the film is a thin sheet of material, such as polypropylene, polystyrene, polyester, polyamide, polyvinylchloride, polyethylene (low density polyethylene, medium density polyethylene and/or high density polyethylene), polyvinylidene chloride, regenerated cellulose, cellulose acetate, and/or combinations thereof. The film material selected may be based on factors such as cost, shelf-life, barrier effectiveness, performance, etc. The film, including the paper-laminated film and any of the embodiments described herein, may have a thickness of less than about 0.25mm (10 mils), particularly from about 0.025mm (1 mil) to about 0.15mm (6 mils). The thickness may be selected based on a variety of factors such as barrier effectiveness, cost, material(s) used, performance characteristics such as transparency and flexibility, etc. The film may be clear or colored. The film may also be printed or plain, and may be flat, patterned or embossed. Also, the film may be laminated with one or more other materials, in addition to paper, such as foil, vinyl or other materials. The film, including the paper-laminated film and any of the embodiments described herein, helps to prevent exposure of the inside of the container 14 to microorganisms, airborne particles and other materials or contaminants.

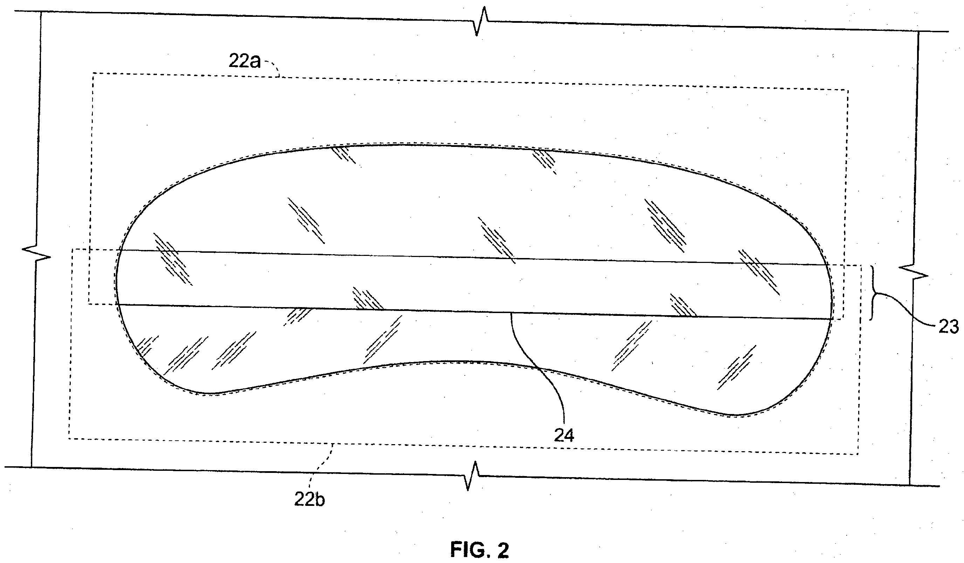

The barrier 22 may be a single sheet of film or may be multi-layered, as shown in FIGS. 1 and 2, as barriers 22a, 22b. The barriers 22, 22a, 22b may comprise the same type of or different materials. The barrier 22, 22a, 22b may also cover all or a portion of the top surface of the container 14, and may include an opening, aperture or slit in the middle of the barrier 22, 22a, 22b for removing the gloves 12. Having additional materials and/or additional layers may provide better protection than a single layer.

As shown in the embodiment of FIG. 2, the barriers 22a, 22b may include at least two overlapping films, including the paper-laminated film and any of the embodiments described herein, that are attached to the container 14. A first overlapping film or barrier 22a may cover a different portion of the opening 18 of the container 14 than a second overlapping film or barrier 22b. The barriers 22a, 22b may be positioned such that the barriers 22a, 22b are adjacent and overlap at an area corresponding approximately to the center of the opening 18, other locations other than the center of the opening 18. Having the overlapping portions 23 at or near the center of the opening 18 may allow for larger areas for users to insert their hands to obtain one or more gloves 12, which may make removing the user's hand and glove easier. The overlapping portion 23 of the barriers 22a, 22b creates a slit 24 which allows a user to insert his or her hand through the slit 24 and remove a glove(s) 12 from the container 14. The barriers 22a, 22b may include a film, a piece of paper laminated with film or any type of flexible material that is suitable for providing or acting as a barrier.

Thus, one method for making the packaging 10 for gloves 12 includes providing a container 14 having a cavity 16 for holding the gloves 12 and an opening 18 for removing the gloves 12 from the container 14. Once a barrier, such as barrier 22, 22a, 22b, is provided which includes the antimicrobial material, a suitable method for attaching the barrier 22, 22a, 22b can be used to cover at least a portion of the opening 18 of the container 14. In some embodiments, the barrier 22, 22a, 22b includes overlapping films or barriers that cover the opening 18 of the container 14 and form the slit 24 for removal of the gloves 12 from the container 14.

In the embodiments shown in FIGS. 1 and 2, the barriers 22, 22a, 22b arc attached to an inside surface of the top portion of the container 14. The barriers 22, 22a, 22b may be attached to the container 14 via adhesive material, glue, heat bonding, mechanical bonding, such as staples, and other suitable modes of attachment. The barriers 22, 22a, 22b may be attached along one or more peripheries, or other suitable area, of the top of the container 14. The barriers 22, 22a, 22b themselves may be made according to various methods, including extrusion, extrusion coating, co-extrusion or calendaring, or other suitable methods for making films. Extrusion, for example, is one of the most common and inexpensive methods for making a film.

The barrier 22, 22a, 22b of the embodiments described herein may include an antimicrobial material(s). The antimicrobial material(s) may include, but is/are not limited to, silver-based antimicrobial materials, copper-based antimicrobial materials, chlorhexidene gluconate, benzalkonium chloride, monoquaternary and polyquaternary ammonium salt-based antimicrobial materials (including covalent bonded quaternary ammonium salt (QAS)), biguanide-based antimicrobials such as polyhexamethylene biguanide (PHMB), triclosan, zinc pyrithionc, isothiazolinone-based antimicrobials, 10,10'-oxybisphenoxarsinc-based (OPBA) antimicrobials, peptide-based antimicrobials, natural antimicrobials such as hops extract, honey and chitosan-based antimicrobials, and any combinations thereof. The antimicrobial material may be selected based on a variety of factors, such as an efficacy requirement (percent of reduction), time to kill, antimicrobial spectrum, i.e., how broadly the antimicrobial material can kill bacteria, or other viruses, mold, fungi, etc. The amount of antimicrobial material used may depend on the specific antimicrobial material used, as different antimicrobial materials will require different levels for effectiveness. Thus, the amount of antimicrobial material needed will vary, but each antimicrobial material will have an antimicrobially effective level.

The antimicrobial material may be added to the barrier 22, 22a, 22b according to different methods that include, but are not limited to, spraying, coating, mixing with a polymer before extrusion, or other methods suitable to result in an application or addition of the antimicrobial material to the barrier 22, 22a, 22b. The particular method chosen may depend on the type of manufacturing process being used to make the barrier 22, 22a, 22b, the end use of the product, cost and other relevant factors. In some embodiments, spraying may be the most cost effective method. In other embodiments, the antimicrobial material may be added to the barrier 22, 22a, 22b by mixing the antimicrobial material with the barrier material before extrusion. Mixing may be advantageous as it does not require additional steps in the manufacturing process. All of these methods provide for the antimicrobial material to be included on the surface of the barrier 22, 22a, 22b, distributed within the barrier 22, 22a, 22b, or both.

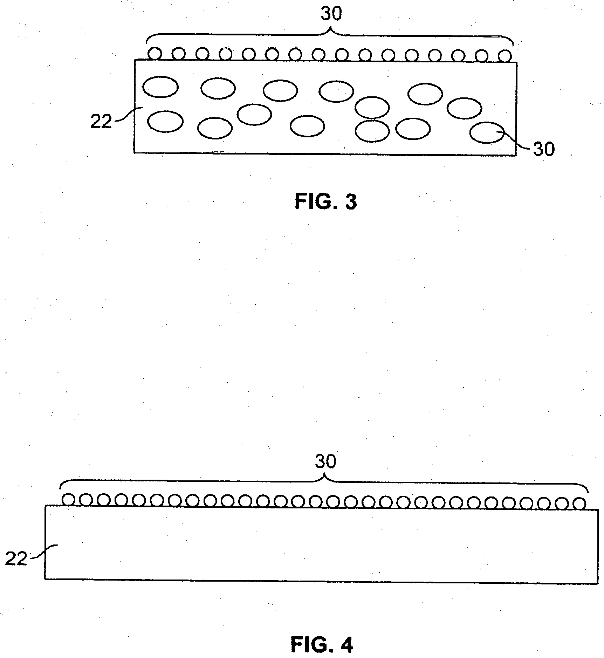

As shown in FIG. 3, the antimicrobial material 30 may be uniformly added to the surface of and incorporated within the barrier 22, 22a, 22b such that once a microorganism comes in contact with the barrier 22, 22a, 22b, it comes into contact with the antimicrobial material 30 and is killed. While it is possible to add the antimicrobial material 30 to only a portion of the barrier 22, 22a, 22b, such as coating the surface of the barrier 22, 22a, 22b with the antimicrobial material 30 as shown in FIG. 4, it is generally more economical and effective to treat the whole barrier 22, 22a, 22b (FIG. 3). In some embodiments, the antimicrobial material 30 distributed within the barrier 22, 22a, 22b may migrate to the surface of the barrier 22, 22a, 22b once the antimicrobial material 30 on the surface is consumed.

Thus, the antimicrobial material 30 that is added to the barrier 22, 22a, 22b destroys the microorganisms and pathogens that may come in contact with the barrier 22, 22a, 22b and thus reduces and/or eliminates the amount of microorganisms that may be deposited on the disposable gloves 12 housed within the container 14. The antimicrobial material 30 in combination with the barrier 22, 22a, 22b creates a contaminant-free zone on the packaging 10 that assists in reducing or eliminating patients and healthcare workers' exposure to potentially infectious and harmful microorganisms and contaminants. Additionally, the barrier 22, 22a, 22b prevents or reduces the occurrence of airborne particles and other materials or contaminants that may contaminate the gloves prior to removal from the packaging.

The following numbered paragraphs set out various aspects and optional features of the packaging for gloves disclosed herein:
1. A packaging for gloves, the packaging comprising:
   a container having a cavity for holding the gloves, the container having an opening for removing the gloves from the container; and
   a barrier positioned to cover at least a portion of the opening of the container, the barrier including an antimicrobial material.
2. The packaging of paragraph 1, wherein the barrier comprises a film.
3. The packaging of paragraph 1, wherein the barrier comprises at least two overlapping films.
4. The packaging of paragraph 1, wherein the barrier comprises polypropylene, polystyrene, polyester, polyamide, polyvinylchloride, polyethylene, polyvinylidene chloride, regenerated cellulose, cellulose acetate and combinations thereof.
5. The packaging of paragraph 1, wherein the antimicrobial material comprises silver-based antimicrobial materials, copper-based antimicrobial materials, chlorhexidene gluconate, benzalkonium chloride, monoquaternary and polyquaternary ammonium salt-based antimicrobial materials, biguanide-based antimicrobials such as polyhexamethylene biguanide, triclosan, zinc pyrithione, isothiazolinone-based antimicrobials, 10,10'-oxybisphenoxarsine-based antimicrobials, peptide-based antimicrobials, natural antimicrobials such as hops extract, honey and chitosan-based antimicrobials, and combinations thereof.
6. The packaging of paragraph 1, wherein the antimicrobial material is added to the barrier by spraying, coating or mixing with the barrier before extrusion.
7. The packaging of paragraph 1, wherein the barrier has a thickness of less than about 10 mils.
8. The packaging of paragraph 7, wherein the barrier has a thickness of from about 1 mil to about 6 mils.
9. The packaging of paragraph 1, wherein the barrier is embossed.
10. The packaging of paragraph 1, wherein the barrier includes a film with a second material.
11. The packaging of paragraph 10, wherein the second material is paper.
12. The packaging of paragraph 1, wherein the barrier is patterned.
13. The packaging if paragraph 1, wherein the gloves are disposable gloves.
14. A method for making a packaging for gloves, the method comprising:
   providing a container having a cavity for holding the gloves, the container having an opening for removing the gloves from the container;
   providing at least a first barrier having an antimicrobial material; and
   attaching the first barrier to the container such that the barrier at least partially covers a portion of the opening of the container.
15. The method of paragraph 14, further comprising providing at least a second barrier having the same or different antimicrobial material as the first barrier.
16. The method of paragraph 15, further comprising attaching the second barrier to the container such that the second barrier covers a different portion of the opening of the container.
17. The method of paragraph 16, wherein the first barrier is adjacent to and at least partially overlaps the second barrier.
18. The method of paragraph 17, wherein the first barrier and the second barrier form a slit from which the disposable gloves may be removed.
19. The method of paragraph 14, wherein the antimicrobial material comprises silver-based antimicrobial materials, copper-based antimicrobial materials, chlorhexidene gluconate, benzalkonium chloride, monoquaternary and polyquaternary ammonium salt-based antimicrobial materials, biguanide-based antimicrobials such as polyhexamethylene biguanide, triclosan, zinc pyrithione, isothiazolinone-based antimicrobials, 10,10'-oxybisphenoxarsine-based antimicrobials, peptide-based antimicrobials, natural antimicrobials such as hops extract, honey and chitosan-based antimicrobials, and combinations thereof.
20. The method of paragraph 14, wherein the antimicrobial material is added to the first barrier by spraying, coating or mixing with the first barrier before extrusion.
21. The method of paragraph 14, wherein the gloves are disposable gloves.
22. A container for holding a plurality of gloves, the container having a body portion and an opening, the opening having an antimicrobial film covering the opening, the antimicrobial film including an aperture to facilitate removal of the gloves from the opening of the container.
23. The container of paragraph 22, wherein the antimicrobial film comprises silver-based antimicrobial materials, copper-based antimicrobial materials, chlorhexidene gluconate, benzalkonium chloride, monoquaternary and polyquaternary ammonium salt-based antimicrobial materials, biguanide-based antimicrobials such as polyhexamethylene biguanide, triclosan, zinc pyrithione, isothiazolinone-based antimicrobials, 10,10'-oxybisphenoxarsine-based antimicrobials, peptide-based antimicrobials, natural antimicrobials such as hops extract, honey and chitosan-based antimicrobials, and combinations thereof.
24. The container of paragraph 22, wherein the antimicrobial film is multi-layered.
25. The container of paragraph 22, wherein the gloves are disposable gloves.

## Claims

1. A packaging (10) for disposable medical gloves (12), the packaging comprising:
a paper container (14) having a cavity (16) configured to hold a plurality of medical gloves (12), the paper container (14) having an opening (18) through which the medical gloves (12) are removable from the paper container (14);
a first barrier (22a) attached to the paper container (14) to cover at least a first portion of the opening (18) of the paper container (14), the first barrier (22a) including a first antimicrobial material; and
a second barrier (22b) attached to the paper container (14) to cover at least a second portion of the opening (18) different from the first portion, the second barrier (22b) having a second antimicrobial material,
**characterized in that**
the first barrier (22a) is adjacent to and at least partially overlaps the second barrier (22b), the first and second barriers (22a, 22b) forming a slit (24) through which the medical gloves (12) are removable from the paper container (14).

2. The packaging of claim 1, wherein the second antimicrobial material of the second barrier (22b) is different from the first antimicrobial material of the first barrier (22a).

3. The packaging of claims 1 or 2, wherein the first and second barriers (22a, 22b) comprise at least two overlapping films.

4. The packaging of claims 1 to 3, wherein the first and second barriers (22a, 22b) each comprises polypropylene, polystyrene, polyester, polyamide, polyvinylchloride, polyethylene, polyvinylidene chloride, regenerated cellulose, or cellulose acetate, or any combination thereof.

5. The packaging of claims 1 to 4, wherein the first and second antimicrobial materials (30) each comprises silver-based antimicrobial materials (30), copper-based antimicrobial materials (30), chlorhexidene gluconate, benzalkonium chloride, monoquaternary and polyquaternary ammonium salt-based antimicrobial materials (30), biguanide-based antimicrobials such as polyhexamethylene biguanide, triclosan, zinc pyrithione, isothiazolinone-based antimicrobials, 10,10'-oxybisphenoxarsine-based antimicrobials, peptide-based antimicrobials, natural antimicrobials such as hops extract, or honey and chitosan-based antimicrobials, or any combination thereof.

6. The packaging of claims 1 to 5, wherein the first and second antimicrobial materials (30) are added to the barriers (22a, 22b) by spraying, coating, or mixing with the barrier before extrusion, or any combination thereof.

7. The packaging of claims 1 to 6, wherein the first and second barriers (22a, 22b) each has a thickness of less than about 0.25 mm (10 mils).

8. The packaging of claim 7, wherein the first and second barriers (22a, 22b) each has a thickness of from about 0.025 mm (1 mil) to about 0.15 mm (6 mils).

9. The packaging of claims 1 to 8, further comprising a segment (20) removably attached to the paper container (14), the segment (20) covering the opening (18).

10. The packaging of claims 1 to 9, wherein the first and second barriers (22a, 22b) comprise different materials.

11. A. method for making a packaging (10) for disposable medical gloves (12), the method comprising:
providing a paper container (14) having a cavity (16) configured to hold a plurality of medical gloves (12), the paper container (14) having an opening (18) through which the medical gloves (12) are removable from the paper container (14);
attaching a first barrier (22a) having a first antimicrobial material to the paper container (14) such that the first barrier (22a) covers at least a first portion of the opening (18) of the paper container (14); and
attaching a second barrier (22b) having a second antimicrobial material to the paper container (14) such that the second barrier (22b) covers at least a second portion of the opening (18) different from the first portion,
**characterized in that**
the first barrier (22a) is adjacent to and at least partially overlaps the second barrier (22b), the first and second barriers (22a, 22b) forming a slit (24) through which the medical gloves (12) are removable from the paper container (14).

12. The method of claim 11, wherein the second antimicrobial material of the second barrier (22b) is different from the first antimicrobial material of the first barrier (22a).

13. The method of claims 11 or 12, wherein the first and second barriers (22a, 22b) are attached to an inside surface of the paper container (14) via an adhesive material.

14. The method of claims 11 to 13, wherein the first barrier (22a) and the second barrier (22b) comprise at least two overlapping films.

15. The method of claims 11 to 14, wherein the first barrier (22a) and the second barrier (22b) each has a thickness of less than about 0.25 mm (10 mils).

## Patentansprüche

1. Verpackung (10) für medizinische Einweghandschuhe (12), wobei die Verpackung Folgendes umfasst:
einen Papierbehälter (14) mit einem Hohlraum (16), welcher konfiguriert ist, um eine Vielzahl medizinischer Handschuhe (12) zu enthalten, wobei der Papierbehälter (14) eine Öffnung (18) aufweist, durch welche die medizinischen Handschuhe (12) aus dem Papierbehälter (14) entnehmbar sind;
- eine erste Trennfläche (22a), welche am Papierbehälter (14) befestigt ist, um zumindest einen ersten Teil der Öffnung (18) des Papierbehälters (14) zu bedecken, wobei die erste Trennfläche (22a) ein erstes antimikrobielles Material umfasst; sowie
- eine zweite Trennfläche (22b), welche am Papierbehälter (14) befestigt ist, um zumindest einen zweiten Teil der Öffnung (18), der sich von dem ersten Teil unterscheidet, zu bedecken, wobei die zweite Trennfläche (22b) ein zweites antimikrobielles Material umfasst,
**dadurch gekennzeichnet, dass**
die erste Trennfläche (22a) an die zweite Trennfläche (22b) angrenzt und diese zumindest teilweise überlappt , wobei die erste und zweite Trennfläche (22a, 22b) einen Schlitz (24) ausbilden, durch welchen die medizinischen Handschuhe (12) aus dem Papierbehälter (14) entnehmbar sind.

2. Verpackung nach Anspruch 1, worin sich das zweite antimikrobielle Material der zweiten Trennfläche (22b) von dem ersten antimikrobiellen Material der ersten Trennfläche (22a) unterscheidet.

3. Verpackung nach Anspruch 1 oder 2, worin die erste und zweite Trennfläche (22a, 22b) zumindest zwei überlappende Folien umfassen.

4. Verpackung nach den Ansprüchen 1 bis 3, worin die erste und zweite Trennfläche (22a, 22b) jeweils Polypropylen, Polystyrol, Polyester, Polyamid, Polyvinylchlorid, Polyethylen, Polyvinylidenchlorid, regenerierte Cellulose oder Celluloseacetat oder eine beliebige Kombination daraus umfassen.

5. Verpackung nach den Ansprüchen 1 bis 4, worin das erste und das zweite antimikrobielle Material (30) jeweils silberbasierte antimikrobielle Materialien (30), kupferbasierte antimikrobielle Materialen (30), Chlorhexidingluconat, Benzalkoniumchlorid, monoquaternäre und polyquaternäre Ammoniumsalz-basierte antimikrobielle Materialien (30), biguanidbasierte antimikrobielle Substanzen wie z. B. Polyhexamethylenbiguanid, Triclosan, Zinkpyrithion, isothiazoinonbasierte antimikrobielle Substanzen, 10,10'-0xybisphenoxarsin-basierte antimikrobielle Substanzen, peptidbasierte antimikrobielle Substanzen, natürliche antimikrobielle Substanzen wie z. B. Hopfenextrakt oder Honig sowie chitosanbasierte antimikrobielle Substanzen oder eine beliebige Kombination daraus umfassen.

6. Verpackung nach den Ansprüchen 1 bis 5, worin das erste und zweite antimikrobielle Material (30) durch Aufsprühen, Beschichten oder Vermischen mit der Trennfläche vor der Extrusion, oder durch eine beliebige Kombination daraus zu den Trennflächen (22a, 22b) hinzugefügt werden,

7. Verpackung nach den Ansprüchen 1 bis 6, worin die erste und zweite Trennfläche (22a, 22b) jeweils eine Dicke von weniger als etwa 0,25 mm (10 Tausendstel Zoll) aufweisen.

8. Verpackung nach Anspruch 7, worin die erste und zweite Trennfläche (22a, 22b) jeweils eine Dicke von etwa 0,025 mm (1 Tausendstel Zoll) bis etwa 0,15 mm (6 Tausendstel Zoll) aufweisen.

9. Verpackung nach den Ansprüchen 1 bis 8, welche ferner ein Segment (20) umfasst, das abnehmbar am Papierbehälter (14) befestigt ist, wobei das Segment (20) die Öffnung (18) bedeckt.

10. Verpackung nach den Ansprüchen 1 bis 9, worin die erste und zweite Trennfläche (22a, 22b) unterschiedliche Materialien umfassen.

11. Verfahren zur Herstellung einer Verpackung (10) für medizinische Einweghandschuhe (12), wobei das Verfahren Folgendes umfasst:
Bereitstellen eines Papierbehälters (14) mit einem Hohlraum (16), welcher konfiguriert ist, um eine Vielzahl medizinischer Handschuhe (12) zu enthalten, wobei der Papierbehälter (14) eine Öffnung (18) aufweist, durch welche die medizinischen Handschuhe (12) aus dem Papierbehälter (14) entnehmbar sind;
Befestigen einer ersten Trennfläche (22a), welche ein erstes antimikrobielles Material aufweist, am Papierbehälter (14), sodass die erste Trennfläche (22a) zumindest einen ersten Teil der Öffnung (18) des Papierbehälters (14) bedeckt; und
Befestigen einer zweiten Trennfläche (22b), welche ein zweites antimikrobielles Material aufweist, am Papierbehälter (14), sodass die zweite Trennfläche (22b) zumindest einen zweiten Teil der Öffnung (18) bedeckt, der sich vom ersten Teil unterscheidet,
**dadurch gekennzeichnet, dass**
die erste Trennfläche (22a) an die zweite Trennfläche (22b) angrenzt und diese teilweise überlappt, wobei die erste und zweite Trennfläche (22a, 22b) einen Schlitz (24) ausbilden, durch welchen die medizinischen Handschuhe (12) aus dem Papierbehälter (14) entnehmbar sind.

12. Verfahren nach Anspruch 11, worin sich das zweite antimikrobielle Material der zweiten Trennfläche (22b) vom ersten antimikrobiellen Material der ersten Trennfläche (22a) unterscheidet.

13. Verfahren nach Anspruch 11 oder 12, worin die erste und zweite Trennfläche (22a, 22b) mittels eines Haftmaterials an einer Innenoberfläche des Papierbehälters (14) befestigt werden.

14. Verfahren nach den Ansprüchen 11 bis 13, worin die erste Trennfläche (22a) und die zweite Trennfläche (22b) zumindest zwei überlappende Folien umfassen.

15. Verfahren nach den Ansprüchen 11 bis 14, worin die erste Trennfläche (22a) und die zweite Trennfläche (22b) jeweils eine Dicke von weniger als etwa 0,25 mm (10 Tausendstel Zoll) aufweisen.

## Revendications

1. Emballage (10) pour des gants médicaux jetables (12), l'emballage comprenant :
un conteneur en papier (14) ayant une cavité (16) configurée pour contenir une pluralité de gants médicaux (12), le conteneur en papier (14) ayant une ouverture (18) à travers laquelle les gants médicaux (12) peuvent être retirés du conteneur en papier (14) ;
une première barrière (22a) reliée au conteneur en papier (14) pour recouvrir au moins une première partie de l'ouverture (18) du conteneur en papier (14), la première barrière (22a) comprenant un premier matériau antimicrobien ; et
une seconde barrière (22b) reliée au conteneur en papier (14) pour recouvrir au moins une seconde partie de l'ouverture (18) différente de la première partie, la seconde barrière (22b) ayant un second matériau antimicrobien,
**caractérisé en ce que**
la première barrière (22a) est adjacente à la seconde barrière (22b) et chevauche au moins partiellement celle-ci, les première et seconde barrières (22a, 22b) formant une fente (24) à travers laquelle les gants médicaux (12) peuvent être retirés du conteneur en papier (14).

2. Emballage selon la revendication 1, dans lequel le second matériau antimicrobien de la seconde barrière (22b) est différent du premier matériau antimicrobien de la première barrière (22a).

3. Emballage selon les revendications 1 ou 2, dans lequel les première et seconde barrières (22a, 22b) comprennent au moins deux films qui se chevauchent.

4. Emballage selon les revendications 1 à 3, dans lequel les première et seconde barrières (22a, 22b) comprennent chacune du polypropylène, polystyrène, polyester, polyamide, chlorure de polyvinyle, polyéthylène, chlorure de polyvinylidène, cellulose régénérée, ou acétate de cellulose, ou toute combinaison de ceux-ci.

5. Emballage selon les revendications 1 à 4, dans lequel les premier et second matériaux antimicrobiens (30) comprennent chacun des matériaux antimicrobiens à base d'argent (30), matériaux antimicrobiens à base de cuivre (30), gluconate de chlorhexidène, chlorure de benzalkonium, matériaux antimicrobiens à base de sel d'ammonium monoquaternaire et polyquaternaire (30), composés antimicrobiens à base de biguanide tels que biguanide de polyhexaméthylène, triclosan, pyrithione de zinc, composés antimicrobiens à base d'isothiazolinone, composés antimicrobiens à base de 10,10'-oxybisphénoxarsine composés antimicrobiens à base de peptide, composés antimicrobiens naturels tels que de l'extrait de houblon, ou composés antimicrobiens à base de miel ou de chitosan, ou toute combinaison de ceux-ci.

6. Emballage selon les revendications 1 à 5, dans lequel les premier et second matériaux antimicrobiens (30) sont ajoutés aux barrières (22a, 22b) par pulvérisation, revêtement, ou mélange avec la barrière avant extrusion, ou toute combinaison de ceux-ci.

7. Emballage selon les revendications 1 à 6, dans lequel les première et seconde barrières (22a, 22b) ont chacune une épaisseur inférieure à environ 0,25 mm (10 mils).

8. Emballage selon la revendication 7, dans lequel les première et seconde barrières (22a, 22b) ont chacune une épaisseur comprise entre environ 0,025 mm (1 mil) et environ 0,15 mm (6 mils).

9. Emballage selon les revendications 1 à 8, comprenant en outre un segment (20) fixé de manière amovible au conteneur en papier (14), le segment (20) recouvrant l'ouverture (18).

10. Emballage selon les revendications 1 à 9, dans lequel les première et seconde barrières (22a, 22b) comprennent des matériaux différents.

11. Procédé pour fabriquer un emballage (10) pour des gants médicaux jetables (12), le procédé comprenant les étapes consistant à :
fournir un conteneur en papier (14) ayant une cavité (16) configurée pour contenir une pluralité de gants médicaux (12), le conteneur en papier (14) ayant une ouverture (18) à travers laquelle les gants médicaux (12) peuvent être retirés du conteneur en papier (14) ;
fixer une première barrière (22a) ayant un premier matériau antimicrobien sur le conteneur en papier (14) de telle sorte que la première barrière (22a) recouvre au moins une première partie de l'ouverture (18) du conteneur en papier (14) ; et
fixer une seconde barrière (22b) ayant un second matériau antimicrobien sur le conteneur en papier (14) de telle sorte que la seconde barrière (22b) recouvre au moins une seconde partie de l'ouverture (18) différente de la première partie,
**caractérisé en ce que**
la première barrière (22a) est adjacente à la seconde barrière (22b) et recouvre au moins partiellement celle-ci, les première et seconde barrières (22a, 22b) formant une fente (24) à travers laquelle les gants médicaux (12) peuvent être retirés du conteneur en papier (14).

12. Procédé selon la revendication 11, dans lequel le second matériau antimicrobien de la seconde barrière (22b) est différent du premier matériau antimicrobien de la première barrière (22a).

13. Procédé selon les revendications 11 ou 12, dans lequel les première et seconde barrières (22a, 22b) sont fixées à une surface intérieure du conteneur en papier (14) par l'intermédiaire d'un matériau adhésif.

14. Procédé selon les revendications 11 à 13, dans lequel la première barrière (22a) et la seconde barrière (22b) comprennent au moins deux films qui se chevauchent.

15. Procédé selon les revendications 11 à 14, dans lequel la première barrière (22a) et la seconde barrière (22b) ont chacune une épaisseur inférieure à environ 0,25 mm (10 mils).
